(19) Europäisches Patentamt / European Patent Office / Office européen des brevets

(11) **EP 4 623 830 A1**

(12) **EUROPEAN PATENT APPLICATION**

(43) Date of publication:
**01.10.2025 Bulletin 2025/40**

(21) Application number: **24167840.8**

(22) Date of filing: **29.03.2024**

(51) International Patent Classification (IPC):
**A61B 6/03** (2006.01) **A61B 6/40** (2024.01)
**A61B 6/00** (2024.01)

(52) Cooperative Patent Classification (CPC):
**A61B 6/032; A61B 6/4021; A61B 6/405;**
**A61B 6/482**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **DAERR, Heiner**
**Eindhoven (NL)**
• **BONTUS, Claas**
**Eindhoven (NL)**

(74) Representative: **Philips Intellectual Property &
Standards
High Tech Campus 52
5656 AG Eindhoven (NL)**

(54) **METHOD FOR PERFORMING SPECTRAL COMPUTED TOMOGRAPHY IMAGING, COMPUTER PROGRAM, COMPUTED TOMOGRAPHY SYSTEM, USE OF A FLYING FOCAL SPOT TECHNOLOGY**

(57) A method for performing spectral computed tomography imaging, the method comprising the following steps: consecutively operating the x-ray source (4) at a first peak kilovoltage over a time frame covering at least part of a first and a second integration period of an x-ray detector; switching the output of the x-ray source (4) from the first peak kilovoltage to a second peak kilovoltage; consecutively operating the x-ray source (4) at the second peak kilovoltage over a time frame covering at least part of a third and a fourth integration period of the x-ray detector; switching the output of the x-ray source (4) from the second peak kilovoltage to the first peak kilovoltage; wherein, during each integration period, a focal spot configuration is applied to the x-ray source (4), to enable an improved overlapping of x-ray beams (1, 2).

FIG. 5

## Description

FIELD OF THE INVENTION

The invention relates to a method for performing spectral computed tomography imaging, a corresponding computer program, a computed tomography system, and a use of a flying focal spot technology.

BACKGROUND OF THE INVENTION

Spectral computed tomography (CT) imaging is a variant of computed tomography imaging that uses different x-ray spectra to differentiate between different materials. This technique makes use of the fact that the x-ray attenuation tends to have a different energy- dependence for different materials. When using two different energy spectra, spectral computed tomography is also referred to as dual-energy computed tomography. Currently, using two different energy spectra is the most common form of spectral computed tomography imaging. However, in principle, spectral computed tomography imaging is not necessarily limited to using only two different spectra and can also be applied with more than two spectra.

One way of applying spectral computed tomography is using two different x-ray sources and detectors, i.e. two combinations of x-ray source and detector. With each x-ray source, the tube voltage and current, and therefore the generated x-ray spectrum, is individually adjusted. However, there are a few disadvantages connected to this approach. For example, additional hardware requirements that are connected to the dual source approach can pose a problem and lead to increased hardware costs. Also, limited space in the CT gantry can be problem when using two x-ray sources and detectors. Furthermore, since there is typically an angular offset between the two source-detector combinations of 90°, a relatively large time delay between the measurements of the same location with different spectra occurs, which can in particular be problematic when patient movement is involved.

An alternative approach to realizing spectral computed tomography is fast kVp switching CT. The peak kilovoltage (short: kVp - may also be called "kilovoltage peak") refers to the maximum voltage that is applied to an x-ray tube in order to generate the x-rays. In early approaches, the applied voltage was typically varied cyclically and was defined by the peak amplitude, hence the denotation peak kilovoltage. If a mostly constant potential is applied, the peak kilovoltage and a steady-state kilovoltage can be identical. For generating the x-rays in CT, typically, electrons are emitted from a heated cathode and accelerated by the applied voltage (kilovoltage) towards an anode (corresponding to an emission current). While the electrons are stopped in the anode, x-rays are produced with an x-ray spectrum that has a maximum photon energy being proportional to the applied peak kilovoltage and that further depends on the anode material. Fast kVp switching CT is based on using a single x-ray source and quickly alternating the tube acceleration voltage between two distinct kVp levels. A typical approach is to switch the kVp at each integration period (IP), such that subsequent integration periods have different kVp levels. Hence, data acquisition may be regarded as low kVp and high kVp scans with interleaved angular positions.

One limitation of kVp switching CT is the switching speed of the applied voltages. In particular, the ramp down speed from the higher kVp value to the lower kVp value is limited, since it is typically accomplished by a discharge of the capacitance between the anode and the cathode via the emission current. The emission current may also be referred to as anode current or tube current. The emission current defines the amount of x-ray radiation that is emitted from the x-ray source - a larger emission current leads to a larger amount of radiation. Hence, the radiation dose that is applied on a patient is typically controlled by the emission current. Patient safety requires to limit the radiation dose. Thus, when a low emission current is required, the required time to switch between the different kVp values may be problem. In particular when a fast scan is required, the switching time (in particular from high kVp to low kVp) may become a great fraction of the whole integration period. For example, a fast scan may be desirable when movement is involved, such as when moving organs, e.g. a beating heart, and/or a moving patient, in particular a pediatric patient, are involved. For example, a pediatric patient may be moving during the scan while at the same time requiring a low dose. Furthermore, future CT scanners may become capable to measure with increased speed, i.e. having a faster gantry rotation and/or providing more readouts per revolution, thus leading to even shorter IPs. Accordingly, when the switching duration times become relatively large in comparison to the overall switching period of the kVp switching, the spectral performance will likely suffer, because an increased spectral overlap may occur between the spectra during the IP of the low kVp and the high kVp.

OBJECT OF THE INVENTION

It is, therefore, an object of the invention to provide a means to better address the above-mentioned problems. In particular it is desirable to provide a means to enable relatively fast kVp switching CT with relatively low radiation dose without compromising spectral performance too much and/or to improve spectral performance for fast kVp switching CT.

SUMMARY OF THE INVENTION

To better address this object, a method according to claim 1, a computer program according to claim 12, a computed tomography system according to claim 13, and a use according to claim 14 is provided. Advantageous embodiments are set out in the dependent claims. Any features, advantages or alternative embodiments described herein in relation to the claimed method are also applicable to the other claim categories and vice versa.

According to a first aspect of the invention, a method for performing spectral computed tomography imaging with a computed tomography system is provided. The computed tomography system comprises a rotating gantry with an x-ray source and an x-ray detector. The computed tomography (CT) system may be a spectral CT system, in particular a dual energy CT system, that is based on kVp switching. The method comprises the following steps:

(a) consecutively operating the x-ray source at a first peak kilovoltage over a time frame covering at least part of a first integration period of the x-ray detector and at least part of a second integration period of the x-ray detector;

(b) switching the output of the x-ray source from the first peak kilovoltage to a second peak kilovoltage, the second peak kilovoltage being different in peak height from the first peak kilovoltage;

(c) consecutively operating the x-ray source at the second peak kilovoltage over a time frame covering at least part of a third integration period of the x-ray detector and at least part of a fourth integration period of the x-ray detector;

(d) switching the output of the x-ray source from the second peak kilovoltage to the first peak kilovoltage;

(e) periodically continuing the pattern of steps (a) to (d) for at least some integration periods;

wherein, during each integration period, a focal spot configuration is applied to the x-ray source, such that, spatially, the x-ray beam in the first integration period at least partially overlaps with an x-ray beam of a previous integration period, the x-ray beam of the second integration period at least partially overlaps with the x-ray beam of the third integration period, and the x-ray beam of the fourth integration period at least partially overlaps with the x-ray beam of a next integration period. In particular, the method steps may be performed in the given order. Preferably, there may be at least two different focal spot configurations that are applied to improve a spatial overlap of at least some of the x-ray beams of different integration periods with different peak kilovoltage.

The computed tomography system comprises a gantry with an x-ray source and an x-ray detector. The x-ray detector may also be referred to as "detector" within the context of this invention. The x-ray source may also be referred to as "source" within the context of this invention. The gantry may be a CT gantry as generally known in the state of the art. A gantry may in particular be a short ring tunnel that is configured such that a subject or an object (e.g., a patient or part of a patient) can be placed inside the area encompassed by the gantry. The gantry may comprise an angular encoder that outputs a signal indicative of a current angular position. The signal may, for example, be a rectangular signal. Hence, each incremental angular position of the rotating gantry may thus be determined. An integration period is to be understood broadly in the context of this invention. It generally refers to the time, that the detector acquires and integrates signals of one CT projection. The integration period may, for example, also be referred to as integration time, sampling time, or acquisition time of the detector. The angular encoder may be used to set the integration periods of the x-ray detector. In particular, there may be a feedback loop between the angular position, the activation of the x-ray source and the integration periods of the x-ray detector. Thus, the timing of the kilovoltage peaks, of the integration periods and of the angular position may be controlled. The angular encoder and/or the timing may be as known in the state of the art.

For example, the x-ray detector may be a curved x-ray detector that partially surrounds a rotational axis of the gantry and a position that the object or subject to be examined is placed at. The length of the curve of the detector, i.e. the detector width, may, for example, be in the range of 100 mm to 2000 mm, preferably 400mm to 1400 mm, more preferably 800 mm to 1100 mm. The detector may comprise multiple pixels. For example, the pixel size of the detector may be in the order of magnitude of about 1 mm. In some cases, e.g. when using a photon-counting detector or detector that is capable to perform photon-counting, the pixel size may be smaller, such as around 300 $\mu$m. Hence, for example, the pixel size may be in the range of about 100 $\mu$m to about 1,5 mm. However, the invention does not have to be limited to a particular pixel size. A detector readout may be performed for each individual pixel. Optionally multiple pixels, e.g. two or three pixels, may be combined for one readout.

The x-ray source may be an x-ray source that is typically used for computed tomography systems as known in the state of the art. The x-ray source may comprise an x-ray tube, a cathode and an anode. Typically, during operation of the x-ray source, electrons are emitted from the cathode, in particular by heating the cathode, and accelerated through the x-ray tube towards the anode via an applied voltage. The voltage may also be referred to as tube voltage. Thus, a beam of electrons (referred to as electron beam) is travelling through the tube and hitting the anode. When the electrons are stopped in the anode, x-rays are generated. Thereby, an x-ray spectrum is generated, with the maximum x-ray photon energy of the spectrum being proportional to the magnitude of the applied voltage. Typically, the x-rays are emitted in a direction perpendicular to the path of the electron beam. Thus, the direction and the origin of the x-rays may be controlled by the electron beam and the location the electron beam is hitting the anode.

When applying the inventive method, at least a first peak kilovoltage and a second peak kilovoltage are applied. The term peak kilovoltage may also be referred to as kVp or kilovoltage peak. The peak kilovoltage corresponds to the voltage that is applied between the cathode and the anode of the x-ray tube. As is known from kVp switching, the applied voltage may be switched between the first peak kilovoltage and the second peak kilovoltage. When applying a peak kilovoltage, e.g. the first peak kilovoltage or the second peak kilovoltage, the applied voltage is preferably essentially constant. Accordingly, when switching between the first peak kilovoltage and the second peak kilovoltage, the voltage may be switched between two voltage steady states or between two voltage plateaus. The second peak kilovoltage is different in peak height from the first peak kilovoltage. For example, the second peak kilovoltage may be lower than the first peak kilovoltage. Accordingly, when switching between the peak kilovoltages, the level of the plateau is changed. The spectral computed tomography imaging is achieved by switching between at least two different peak kilovoltages, comprising the first peak kilovoltage and the second peak kilovoltage. Hence a kVp switching is performed to realize the spectral computed tomography imaging. The kVp switching may also be referred to as fast kVp switching or rapid kVp switching. In state-of-the-art kVp switching, the voltage is typically switched every integration period (IP). However, the switching speed is limited. Since the voltage depends on the capacitance between the cathode and the anode, switching the voltage will take at least some time that is greater than zero. In particular, a lowering of the voltage is typically achieved by a discharge of the capacitance via the emission current, to decrease the voltage between the cathode and the anode. Hence, there will be a transition time, during which the voltage is adapted. During the transition times, the voltage will be in between the first peak kilovoltage and the second peak kilovoltage. This rise and fall time of the voltage decreases the spectral separation. A good material differentiation typically requires a strong difference between the photon energies and, thus, of the applied voltages, which corresponds to a strong spectral separation. In particular, for short integration periods (i.e., fast switching is required), and low dose applications (i.e., a low emission current is required leading to a slower lowering of the voltage), the transition times may become so large compared to the plateau times that the spectral performance, that depends on a good spectral separation between the first and second peak kilovoltage, may be impaired significantly. For a very low emission current, the discharge time may be in a similar order of magnitude as the duration of an integration period or even exceed it. For example, rise and fall times may be in the order of magnitude of a few 10 $\mu$s (e.g. rise time about 20 $\mu$s and fall time about 40 $\mu$s at 400 mA). In future CT systems, the integration periods may get even smaller related to faster rotation (e.g. 300 rpm = 5 Hz), provide smaller detector pixels, and, at the same time, may provide more readouts per revolution. For example, a typical detector pitch may be about 1 mm in x direction (direction perpendicular to the gantry axis, i.e. perpendicular to the z-direction) and the source to detector distance may be in the range of about 1000mm. This may result in about 3140 readouts per revolution if the angular increment fits the angular detector coverage. In this example, the integration period may be as short as 200ms/3140 ≈ 63 $\mu$s and the sum of the rise and the fall time during switching may reach a great fraction of a single integration period.

Advantageously, the method allows to improve the spectral performance during spectral computed tomography imaging by applying the respective peak kilovoltages not only during one integration period but at least partially during two integration periods. Hence, the first peak kilovoltage is applied during a time frame covering at least part of the first integration period and the second integration period and the second peak kilovoltage is applied during a time frame covering at least part of the third integration period and the fourth integration period. Covering part of an integration period may in particular be understood such, that at some time during the integration period, the transition between the first and the second peak kilovoltage may take place. For example, switching the output of the x-ray source from the first peak kilovoltage to the second peak kilovoltage may start in the second integration period and end in the third integration period or it may start and end entirely in the second integration period or it may start and end entirely in the third integration period. For example, switching the output of the x-ray source from the second peak kilovoltage to the first peak kilovoltage may start in the fourth integration period and end in the next (i.e. fifth) integration period or it may start and end entirely in the fifth integration period or it may start and end entirely in the next integration period. The switching rate between the first and the second peak kilovoltage may thus be reduced, in particular by a factor of two, corresponding to the two integration periods during which the same peak kilovoltage is applied. Hence, the relative duration of the switching/transition times may thus become smaller relative to the duration during which the respective kilovoltage peaks are applied.

The first, second, third, and fourth integration period are to be understood exemplarily. In the context of the invention, it is not necessary to start a scan with the "first integration period". There may be one or several integration periods before the first integration period. The numbering of the integration periods may be understood in relation between the different integration periods. Hence, the second integration period comes after the first integration period, the third integration period comes after the second integration period and the fourth integration period comes after the third integration period. Preferably the integration periods may follow directly one after the other according to the numbering. However, optionally, in some embodiments, there may be one or several additional integration periods in between the numbered integration periods. The general concept of the invention may, in principle, also be performed with this variant.

The pattern of steps (a) to (d) is continued periodically. For example, after the fourth integration period, a fifth integration period may follow that corresponds to the first integration period and a sixth integration period may follow that corresponds to the second integration period, etc. Hence, for example, the x-ray source may be operated at the first peak kilovoltage over a time frame covering at least part of a fifth integration period of the x-ray detector and at least part of a sixth integration period of the x-ray detector. The further steps (b) to (d) may be cycled through accordingly.

While the application of the first and second peak kilovoltages over more than one integration period is advantageous to achieve a better spectral separation, a material differentiation may be achieved to a better degree, when the corresponding x-ray beams of the projection for both energies are as similar as possible. Applying the same voltage over two integration periods may thus have the detrimental effect that the rotation of the gantry between the consecutive application of two different x-ray spectra (corresponding to the first peak kilovoltage and the second peak kilovoltage) is further progressed. Accordingly, the angular difference between the two x-ray beams may be larger, leading to a greater difference in the path of the x-ray beam. It may be possible to somewhat interpolate the different angular positions to artificially compensate for this effect. However, interpolation of the measured intensities from different angular position is an estimation and may produce errors. This effect may advantageously be worked against by the application of the focal spot configuration. In the state of the art, controlling the focal spot is known. In particular, in the state of the art, a method denoted "flying focal spot" is known to be applied in order to increase the resolution of a CT scan. The known technique of controlling the flying focal spot may be used to control the focal spot configuration according to this invention. Generally, the focal spot describes the spot at the anode of the x-ray source where the electrons hit the anode. Since the x-ray beam depends on the focal spot, controlling the focal spot may thus allow to control the x-ray beam. Typically, the x-ray beam is cone-shaped and/or fan-shaped. However, other shapes of the x-ray beam may also be conceivable in some embodiments. The focal spot configuration may in particular comprise the position at which the electrons hit the anode. Hence, the focal spot configuration may comprise the position of the focal spot. The focal spot configuration may comprise the shape and size of the focal spot. Preferably, the shape and size of the focal spot is essentially the same for the focal spot configuration of the first and second peak kilovoltage. In the context of this invention, the focal spot configuration may be numbered according to the corresponding integration period. The focal spot configuration of the first integration period may be denoted as first focal spot configuration. The focal spot configuration of the second integration period may be denoted as second focal spot configuration. The focal spot configuration of the third integration period may be denoted as third focal spot configuration. The focal spot configuration of the fourth integration period may be denoted as fourth focal spot configuration. The focal spot configuration of the next (or fifth) integration period may be denoted as next (or fifth) focal spot configuration. The focal spot configuration is applied such that the x-ray beam in the first integration period at least partially overlaps with an x-ray beam of the previous integration period, the x-ray beam of the second integration period at least partially overlaps with the x-ray beam of the third integration period, and the x-ray beam of the fourth integration period at least partially overlaps with the x-ray beam of a next integration period. The next integration period in particular corresponds to the fifth integration period. In particular, due to the cyclical application of the method steps, the applied peak kilovoltage during the next integration period is the first peak kilovoltage. The previous integration period in particular corresponds to the integration period directly before the first integration period, e.g. the 0th integration period. In particular, due to the cyclical application of the method steps, the applied peak kilovoltage during the previous integration period is the second peak kilovoltage. Advantageously, there may thus be more similar pathways and/or a significant overlap of x-ray beams of different, consecutively applied peak kilovoltages. The overlap is in particular to be understood with respect with respect to the object or subject that is examined. Hence, the corresponding x-ray beams are projected over at least partially the same area. Preferably the peak focal spot configurations are applied such that the overlap is greater than it would be without the application of the focal spot configurations. Due to the consecutive application of the projections, the x-ray beams are typically not applied at the same time. Hence, typically, there will not be a temporal overlap. Advantageously, due to the (partial) overlap, the data from pairs of two different x-ray spectra that are taken consecutively may thus match better spatially, covering (partially) the same area of and/or path through the examined object or subject allowing better material decomposition. Material decomposition is a method for differentiation and quantification of scanned materials that is based on the energy dependence of the materials' attenuation. Material decomposition as such is generally known in the state of the art. For example, the material decomposition may be performed in the projection domain or the image domain of the scanned CT data.

Preferably, the corresponding x-ray beams overlap spatially essentially completely. "Essentially completely" may be understood such that, due to a (small) rotation of the gantry, there may be a slight mismatch of the overlap due to the different angular position of the x-ray source. By application of this feature the same line-integral or at least a very similar line-integral may be measured at each kVp level. Hence, overlapping beams may be advantageous, that a material decomposition can be determined particularly well. For example, there are typically two effects that mainly contribute to the energy-dependent attenuation of the x-ray beam, namely Compton scattering (Compton effect) and the photoelectric effect. The Compton effect depends on the electron density of the material, wherein, typically, the main factor is the electron density. The photoelectric effect, on the other hand, strongly depends on the atomic number of the material. Measuring a basically identical x-ray path through the material twice at different tube voltages (i.e. the first and the second peak kilovoltage) may thus allow to determine both these effects.

According to an embodiment, the focal spot configuration in the third integration period differs from the focal spot configuration in the second integration period such that, spatially, the x-ray beam in the third integration period and the x-ray beam in the second integration period are rotationally shifted towards each other, in particular at least diminishing an angular separation of the two beams that is due to the gantry's rotation. The shift of the two beams may be applied to the other beams accordingly. There may be a pairwise shift of x-ray beams being generated by different peak kilovoltages. Hence, accordingly, the x-ray beam in the first integration period and the x-ray beam in a previous second integration period may be rotationally shifted towards each other. The x-ray beam in the fourth integration period and the x-ray beam in the next (fifth) integration period may be rotationally shifted towards each other. The gantry is typically rotating during a scan, e.g. either stepwise or continuously. This leads to consecutive x-ray beams of consecutive integration periods of the detector covering a different path through the examined object or subject, namely a path that is shifted according to the rotation of the gantry. According to this embodiment, this effect is at least partially compensated for by shifting the otherwise angularly separated x-ray beams towards each other. It may be provided that either one of the two beams are shifted, the other beam being at a standard position or that both beams are shifted. Relevant is in particular, that there is a relative shift between the two beams that leads to a spatial overlap of the beams.

According to an embodiment, the difference between the second focal spot configuration and the third focal spot configuration is such that the angular separation of the two beams that is due to the x-ray source's rotation is essentially compensated. For example, the absolute position of the focal spot of the second focal spot configuration may be the same as the absolute position of the focal spot of the third focal spot configuration. The absolute position of the focal spot may, for example, be defined as a position with respect to the center of rotation of the gantry. The compensation of the two beams may be applied to the other beams accordingly. There may be a pairwise shift of x-ray beams being generated by different peak kilovoltages. Hence via this compensation, the corresponding x-ray beams may overlap spatially essentially completely. Hence, additionally or alternatively, the difference between a previous focal spot configuration and the first focal spot configuration may be such that the angular separation of the two beams that is due to the x-ray source's rotation is essentially compensated. For example, the absolute position of the focal spot of the previous focal spot configuration may be the same as the absolute position of the focal spot of the first focal spot configuration. Additionally or alternatively, the difference between the fourth focal spot configuration and the next focal spot configuration may be such that the angular separation of the two beams that is due to the x-ray source's rotation is essentially compensated. For example, the absolute position of the focal spot of the fourth focal spot configuration may be the same as the absolute position of the focal spot of the next focal spot configuration. This embodiment may advantageously allow a particularly good determination of the material decomposition. In the state of the art, it is sometimes approximately assumed that the both x-ray beams are at the same position, while, in reality, due to the rotation, this is in fact not the case. For example, there may be 3000 different angular views during one rotation, leading to a slight shift between consecutive beams. This discrepancy would be even greater with the inventive method of applying the kVp settings for more than one integration period. In the state of the art, sometimes an interpolation is used to compensate for the actual shift. However, an interpolation is only an estimation and can therefore be slightly wrong at least in some cases. Advantageously, due to compensation via the focal spot this disadvantage of the slight discrepancy can be avoided. In particular disadvantages of an interpolation estimate may be avoidable.

According to an embodiment, the second peak kilovoltage is lower than the first peak kilovoltage, wherein switching the output of the x-ray source from the first peak kilovoltage to the second peak kilovoltage is performed within the second integration period and switching the output of the x-ray source from the second peak kilovoltage to the first peak kilovoltage is performed in the integration period following after the fourth integration period. In other words, the transitions between the two peak kilovoltage are performed during the integration periods of the first peak kilovoltage. Advantageously, the rising and falling of the tube voltage (i.e. the switching of the voltage) are thus applied during the integration periods of the higher first peak kilovoltage and not during the integration periods of the lower peak kilovoltage of the second peak kilovoltage. Typically, a strong signal at both applied energy spectra (corresponding to the first kVp and the second kVp) and a good separation between the two spectra is wanted to allow for a good material decomposition. The x-ray intensity strongly depends on the kVp level and is higher for a higher kVp level. The kVp level during a transition is generally higher than for the lower second peak kilovoltage. Hence, the transition times contains a relatively high flux compared to the lower second peak kilovoltage. The transition times may thus influence the integrated detector signal of the second peak kilovoltage significantly more strongly than the detector integration of the higher first peak kilovoltage. Therefore, the spectral performance may improve by including the transitions in the integration periods of the higher first peak kilovoltage.

According to an embodiment, the second peak kilovoltage is applied continuously essentially over the entirety of the third integration period and the fourth integration period. In particular, the x-ray source is operated at plateau voltage values with transition times between different plateau voltage values, wherein the plateau voltage values comprise the first peak kilovoltage and the second peak kilovoltage, wherein the plateau of the second peak kilovoltage is applied continuously essentially over the entirety of the third integration period and the fourth integration period. In particular, no transition times are in the third integration period and in the fourth integration period. The measured intensity $I$ in an integration period may be described as the intensity measured at the transitions $I_t$ and the intensities at the plateaus $I_p$ (for example, considering a first peak kilovoltage of 140 kVp and a second peak kilovoltage of 80 kVp, $I_{p,80}$ at 80 kVp or $I_{p,140}$ at 140 kVp):

$$I = I_p + I_t$$

In this example, for $I_{p,80}$ the intensity $I_t$ typically contains a relatively high flux as the transition contains only kVp levels larger than 80 kVp. And $I_t$ may become equal or even greater than $I_{p,80}$. For the high integration period, the situation is different as the transitions only contain lower kVp-level and in general $I_t$ will be smaller than $I_p$ (hence, in this example, $I_{p,140}$). Therefore, the spectral performance may improve by including the transitions in the high integration period.

According to an embodiment, the focal spot configuration during the integration periods is controlled by manipulating the electron beam that is used for the x-ray generation of the x-ray source, in particular via electrostatic and/or magnetic fields. In particular, the electron beam may be deflected by the electrostatic and/or magnetic fields. Manipulating the electron beam by electrostatic and/or magnetic fields may be a reliable and relatively easy way to achieve different focal spot configuration. The focal spot configuration may be controlled such, that the relative focal spot position changes from integration period to integration period.

According to an embodiment, the focal spot configuration is controlled via setting the location of the focal spot. In particular the location of the focal spots may be different corresponding to the different angular position of the x-ray source due to the rotation of the gantry between two consecutive different peak kilovoltages, the two consecutive different peak kilovoltages in particular being the first peak kilovoltage and the second peak kilovoltage.

According to an embodiment, the spot at which the electron beam hits the anode of the x-ray source is controlled to be different for the focal spot configurations when operating the x-ray source at the first peak kilovoltage than for the focal spot configurations when operating the x-ray source at the second peak kilovoltage during two integration periods following directly one after the other. This embodiment can be a relatively easy solution to achieve identical or nearly identical line paths of different x-ray spectra following directly one after the other.

According to an embodiment, the x-ray detector comprises multiple angularly arranged detector elements, wherein the angular shift between the focal spot configuration of consecutive integration periods is set to correspond to the angular distance or a multiple of the angular distance between the angular position of neighboring detector elements of the detector. The angular shift may match the angular distance in terms of the involved angles. In some embodiments, the rotational axis may not be exactly in the center between the detector and the focal spot position of the x-ray source. In particular, the distance between the focal spot position and the rotational axis may be greater than the distance between the detector elements and the rotational axis. In this case the distance on the circular arc of the detector between the detector elements may be correspondingly smaller than the distance on the circular arc of the anode around the rotational axis. Hence the focal spot configuration may be adapted to the configuration of the detector elements. This may allow to achieve a better signal, e.g. a better resolution. Optionally, the step size of the gantry's rotation may be adapted to the angular distance of the detector elements. In particular, the angular shift between the focal spot configuration of consecutive integration periods may be set to correspond to the step size of the gantry's rotation.

According to an embodiment, the first peak kilovoltage is in the range of 110 kVp to 1000 kVp, preferably 120 kVp to 200 kVp, more preferably 130 kVp to 150 kVp and/or wherein the second peak kilovoltage is in the range of 30 kVp to 100 kVp, preferably 50 kVp to 95 kVp, more preferably 70 kVp to 90 kVp. Additionally or alternatively, according to an embodiment, a ratio between the first peak kilovoltage and the second peak voltage is at least 1.2, preferably at least 1.5, more preferably at least 1,7 and/or wherein a ratio between first peak kilovoltage and the second peak voltage is at most 2.5, preferably at most 2.2, more preferably at most 2. Additionally or alternatively, according to an embodiment, a difference between the first peak voltage and the second peak volage is in the range of 10 to 200 kVp, preferably 30 to 150 kVp, more preferably 40 to 100 kVp, and most preferably 50 to 80 kVp. In order to achieve a good material decomposition, a large difference in energy (corresponding to a large difference in voltage) is advantageous. On the other hand, voltages that are too low may have the disadvantage of not giving sufficient signal strength, especially after passing through an object, while voltages that are too high may deliver a dose that is too high for many applications with human patients. In some cases, a voltage that is too high may lead to radiation spectra that are mostly not attenuated by the examined material, which may be detrimental for image quality. Hence, the given value ranges may be advantageous in allowing a good material decomposition while also not resulting in radiation doses that are too high.

According to an embodiment, the lengths of the integration periods are each in the range of 20 μs to 1000 μs, preferably 30 μs to 500 μs, more preferably 40 μs to 200 μs. The given ranges may provide both a sufficient signal strength and/or signal-to-noise ratio and be suitable to provide a fast enough scan, e.g. to reduce motion artifacts.

According to a further aspect of the invention, a computer program comprising instructions which, when the program is executed by a control station of a computed tomography system, cause the computed tomography system to carry out the method as described herein. The instructions may comprise any of the method steps and their variants as described herein. The computer program may be stored on a data storage medium, in particular a non-volatile data storage medium. The storage medium may be any computer-readable storage medium. For example, the storage medium may be a solid-state storage medium or an optical storage medium. The storage medium may be a hard-drive, a solid-state-disk, a compact disk, a DVD, a flash storage an online server etc.

According to a further aspect of the invention, A computed tomography system comprising a rotatable gantry with an x-ray source and an x-ray detector and a control station is provided. The control station is configured to let the computed tomography system perform the method as described herein. The computed tomography system may be a spectral computed tomography system, in particular a dual energy computed tomography system, that is based on peak kilovoltage switching, in particular fast peak kilovoltage switching.

According to a further aspect of the invention, a use of a flying focal spot technology in kilovoltage peak switching computed tomography is provided. The flying focal spot mode may be selected such that a relative focal spot position changes from integration period to integration period of the x-ray detector. In particular the flying focal-spot kilovoltage peak switching may be performed such that each focal spot and/or x-ray projection is measured with a low and a high peak kilovoltage. The flying focal spot technology may be applied as described herein with respect to the focal spot configuration, in particular the first and the second focal spot configurations.

According to an embodiment, the flying focal spot technology is used to create and/or increase a spatial overlap of at least two consecutively generated x-ray beams with different peak kilovoltage. Thus, advantageously, via the flying spot technology an overlap and/or an increased overlap of x-ray beam projections may be achieved. The flying focal spot technology may be used to compensate for an angular distance of at least some of the x-ray projections, in particular x-ray projections having different energy spectra, of different integration periods of the computed tomography system's x-ray detector that is caused by the rotation of the computed tomography system's gantry. Thus, a material decomposition when evaluating scanned data may be improved. In comparison, in a single focal spot mode, the angular distance between the same peak kilovoltage levels would be larger and an interpolation of the measured intensities may produce errors.

The features and advantages of different embodiments can be combined. The features and advantages of one aspect of the invention, e.g., the method, may be applied to the other aspects, e.g., computer program, the computed tomography system, and the use, and vice versa.

BRIEF DESCRIPTION OF THE DRAWINGS

The invention shall now be illustrated by means of embodiments with reference to the attached drawings, in which:

> Fig. 1 shows a plot of the tube voltage that may be applied in the x-ray source during a fast kVp switching CT scan, wherein the fast kVp switching is applied according to the state of the art;
> Fig. 2 shows a flow diagram of a method for performing spectral computed tomography imaging according to an embodiment of the invention;
> Fig. 3 shows an exemplary plot of an applied tube voltage according to an embodiment of the invention;
> Fig. 4 shows an exemplary plot of an applied tube voltage according to an embodiment of the invention;
> Fig. 5 shows a schematic representation of adapting the focal spot configuration within a method according to the invention;
> Fig. 6 shows an exemplary plot of an applied tube voltage according to an embodiment of the invention;
> Fig. 7 shows a schematic representation of adapting the focal spot configuration within a method according to the invention, wherein several consecutively applied beams are shown at the same time; and
> Fig. 8 shows a side view of a computed tomography system according to an embodiment of the invention.

DESCRIPTION OF EMBODIMENTS

Throughout the Figures, the same or corresponding features/elements of the various embodiments are designated with the same reference numbers.

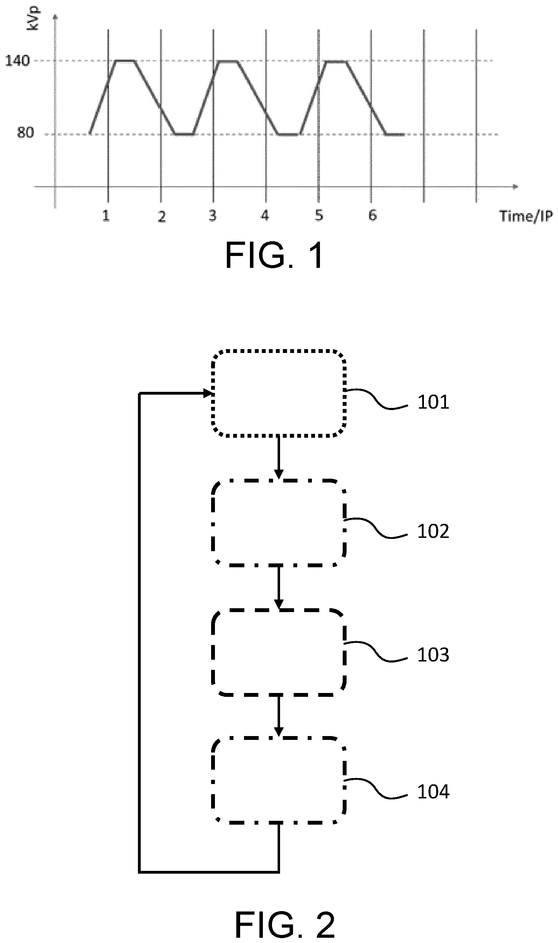

Fast kilovoltage peak (kVp) switching computed tomography (CT) is a method for performing spectral computed tomography imaging. In fast kVp switching CT, a tube voltage of an x-ray source is switched between different voltages in order to generate different x-ray spectra. With the tube voltage, electrons that are emitted from a cathode of the x-ray source are accelerated towards an anode of the x-ray source. Due to the interaction of the electrons with the anodes, x-rays are generated. Fig. 1 shows a plot of the tube voltage that may be applied in the x-ray source during a fast kVp switching CT scan. In this plot, as well as in the plots shown in the following Figures, the voltage is on the vertical axis and the time is on the horizontal axis. The time is plotted in units of integration periods, i.e. the time slots during which the CT detector integrates over a detected signal. In this example, the tube voltage is switched between 80 kVp and 140 kVp. In the plot, there are plateaus that correspond to the two kVp values and rising and falling parts that correspond to the switching/transition of the tube voltage from 80 kVp to 140 kVp and vice versa. Relatively constant plateaus may be achieved by using a generator that keeps the voltage steady and compensates for the voltage drop caused by the discharge by the emission current. Depending on the kVp value different x-ray spectra are generated, wherein a maximum energy of each x-ray spectrum corresponds to the kVp value (i.e., for the voltage plateaus shown here, corresponding to 80 kVp or 140 kVp). The tube voltage is switched in a rate corresponding to the integration periods, which is a typical approach used in the state of the art. As can be seen by the moderately steep switching flanks, the switching speed of the tube voltages is limited. In particular the ramp down from 140 kVp to 80 kVp is rather slow. For this purpose of ramping down, the generator that enables the steady plateaus may be switched off. Nevertheless, the ramping down is limited because it is typically accomplished by a discharge of the capacitance between the anode and the cathode of the x-ray source using the emission current. The emission current, on the other hand governs the radiation dose and, therefore, cannot be increased arbitrarily. For example, the rise and fall times may be in the order of a few 10 μs, such as a rise time of about 20 μs and a fall time of about 40 μs at an emission current of 400 mA. Due to the relatively short integration periods in this example, the sum of the rise and the fall times during switching reaches a great fraction of a single integration period. For example, a CT scanner with fast gantry rotation, such as 300 rpm = 5Hz, and about 3140 readouts per revolution, may lead to integration periods of about 63 μs. Since the detector integrates over the length of the integration period, the effective signal received by the detector will be shifted towards a value that is between the plateaus in each integration period. Consequently, a relatively large overlap exists between the spectra of the low kVp and the high kVp integration periods. This large overlap will limit the spectral performance and the distinction between different materials will be more difficult. This problem is greater the faster the scan is (i.e. having shorter integration periods) and the lower the applied dose is (i.e. using a smaller emission current to reduce the dose, thereby increasing the ramp down time).

Fig. 2 shows a flow diagram of a method for performing spectral computed tomography imaging according to an embodiment of the invention. The spectral computed tomography imaging is performed via a kVp switching with a computed tomography system having a gantry 10 with an x-ray source and an x-ray detector. However, with respect to the variant shown in Fig. 1, the switching pattern is changed.

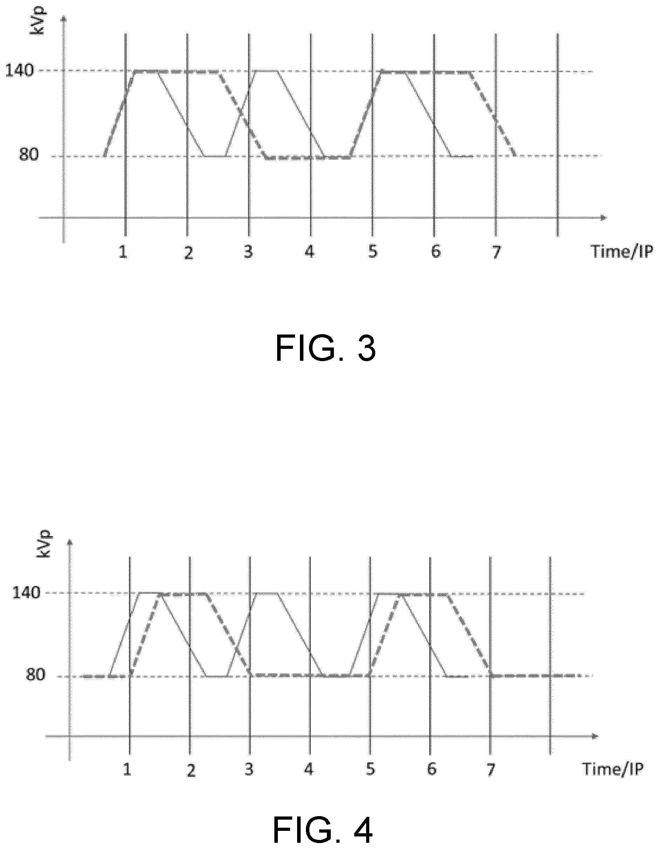

In a first step 101, the x-ray source is operated at a first peak kilovoltage over a time frame covering at least part of a first integration period of the x-ray detector and at least part of a second integration period of the x-ray detector. Preferably the first peak kilovoltage is in the range of 120 kVp to 200 kVp. In the given examples, the first peak voltage is at 140 kVp (see Figs 3, 4 and 6).

In Fig. 3, a corresponding tube voltage signal (dashed line) according to an embodiment of the invention is shown. The first integration period starts at the number 1 in the time axis and lasts up to the number 2, where the second integration period starts. The dashed line shows the course of the voltage according to an embodiment of the invention. The solid line shows, for comparison, a course of the voltage as it may be found similarly in the state of the art, in particular corresponding to the plot shown in Fig. 1. It can be seen that the plateau of the dashed line in Fig. 3 covers part of the first integration period and part of the second integration period. The plateau going from the first integration period to the second integration period corresponds to the first peak kilovoltage. Hence, the durations of the plateaus of the first peak kilovoltage according to the invention (dashed line) are longer than the durations of the corresponding plateaus according to the example from the state of the art (solid line). As indicated by the area of the plot on the left of the first integration period (where a rising flank of the plot is shown in the "0th" integration period), the first integration period does not have to be the first integration period of the plot but it is named "first" integration period for exemplary reasons.

Referring to Fig. 2, in a following step 102, the output of the x-ray source is switched from the first peak kilovoltage to a second peak kilovoltage, the second peak kilovoltage being lower in peak height than the first peak kilovoltage. Preferably the second peak kilovoltage is in the range of 50 kVp to 95 kVp. In the given examples of the Figs 3, 4, 6, the second peak kilovoltage is 80 kVp. In the embodiment shown in Fig. 3, the switching starts in the second integration period and ends within the third integration period.

Referring to Fig. 2, in a following step 103, the x-ray source is consecutively operated at the second peak kilovoltage over a time frame covering at least part of a third integration period of the x-ray detector and at least part of a fourth integration period of the x-ray detector.

In the embodiment shown in Fig. 3, the plateau of the second peak kilovoltage (at 80 kVp) starts during the third integration period after ramping down of the tube voltage (dashed line) is finished and it lasts up to the moment when ramping up the voltage again starts during the fourth integration period. Comparing the dashed line according to the invention with the solid line according to the state of the art, it can be seen that the individual switching times are essentially equal for both voltage signals line, but the plateau times of both the first peak kilovoltage and the second peak kilovoltage are longer for the dashed line according to the invention, because the switching only occurs every second integration period.

Fig. 4 shows a plot of the tube voltage according to another embodiment of the invention. The dashed line shows a plot of the voltage according to this embodiment of the invention and the solid line shows a plot of the voltage according to Fig. 1 for comparison. In this embodiment, represented by the dashed line, the (lower) second peak kilovoltage (plateau of 80 kVp) is applied continuously essentially over the entirety of the third integration period and the fourth integration period. The rising and falling of the voltage is entirely covered by the integration periods of the higher first peak kilovoltage (plateau of 140 kVp). The x-ray intensity strongly depends on the kVp level having a higher intensity at a higher kVp level and, thus, signal intensity of the lower kVp tends to be weaker and can be influenced more strongly by the rising and falling flanks that are naturally higher than the lower plateau. Hence, the spectral separation between the two x-ray spectra may thus be weakened. By providing an embodiment as shown in Fig. 4, wherein the integration periods of the lower second peak kilovoltage essentially only contain the plateau, this problem of weakening the spectral separation due to the rising and falling times of the tube voltage can be reduced.

Referring to Fig. 2, in a following step 104, the output of the x-ray source is switched from the second peak kilovoltage to the first peak kilovoltage.

In the embodiment shown in Fig. 3, this switching back (i.e., the onset of the ramping up) starts during the fourth integration period and ends during the fifth integration period.

In the embodiment shown in Fig. 4, the switching back starts at the beginning of the fifth integration period and also ends during the fifth integration period.

Referring to Fig. 2, the pattern of the method steps 101-104 is cyclically continued during the computed tomography scan as indicated by the arrow from step 104 to step 101.

This continuation is also indicated in the embodiments shown in Figs 3 and 4, where, after the fourth integration period, further integration periods, including a fifth and sixth integration period etc, follow.

During application of the method as shown in the flow diagram of Fig. 2, a focal spot configuration is applied to the x-ray source during each integration period. The focal spot configuration is applied such that, spatially, the x-ray beam 1 in the first integration period at least partially overlaps with an x-ray beam 2 of a previous integration period, the x-ray beam 1 of the second integration period at least partially overlaps with the x-ray beam 2 of the third integration period, and the x-ray beam 2 of the fourth integration period at least partially overlaps with the x-ray beam 1 of a next integration period. In Figs 3, 4 and 6, the previous integration period corresponds to the integration period on the left of the first integration period ("0th" integration period) and the next integration period corresponds to the fifth integration period. For example, the focal spot configuration during the integration periods may be controlled by manipulating the electron beam that is used for the x-ray generation of the x-ray source, in particular via electrostatic and/or magnetic fields.

Preferably the focal spot configuration in the third integration period differs from the focal spot configuration in the second integration period such that, spatially, the x-ray beam 2 in the third integration period and the x-ray beam in the second integration 1 periods are rotationally shifted towards each other, diminishing an angular separation of the two beams that is due to the gantry's rotation. More preferably, the difference between the second focal spot configuration and the third focal spot configuration is such that the angular separation of the two beams that is due to the x-ray source's rotation is essentially compensated. Preferably, the focal spot configuration in the first and in the previous integration periods as well as in the fourth and in the next integration periods differ correspondingly such that there are pairs of x-rays beams based on the first peak kilovoltage and the second peak kilovoltage, respectively that have essentially the same line integrals.

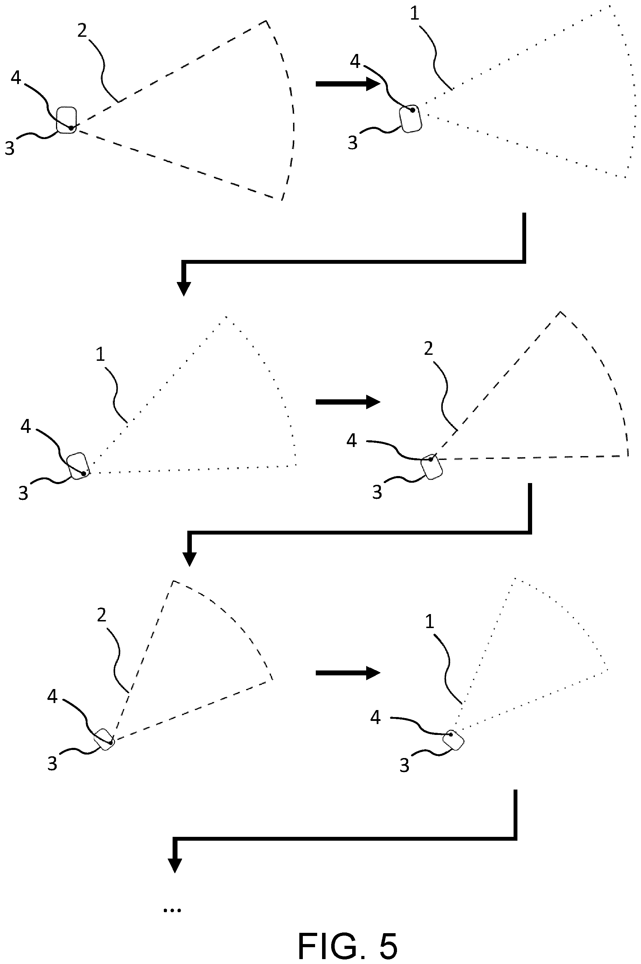

Fig. 5 shows a schematic representation of adapting the focal spot configuration to achieve identical or very similar x-ray beam paths for different x-ray spectra, i.e. x-ray spectra based on the first peak kilovoltage and the second peak kilovoltage. The x-ray beams 1, 2 are represented only schematically and may have a different shape. For example, the x-ray beams 1, 2 may have a greater fan angle. In this representation, the fan angle is kept relatively small to allow for a better overview and easier differentiation between the different beams. While the x-ray source 3 is rotating around a center of rotation, a focal spot configuration is applied by shifting the focal spot 4 of the x-ray beams 1, 2. The dotted lines represent x-ray beams 1 generated via the first peak kilovoltage. The dashed lines represent x-ray beams 2 generated via the second peak kilovoltage.

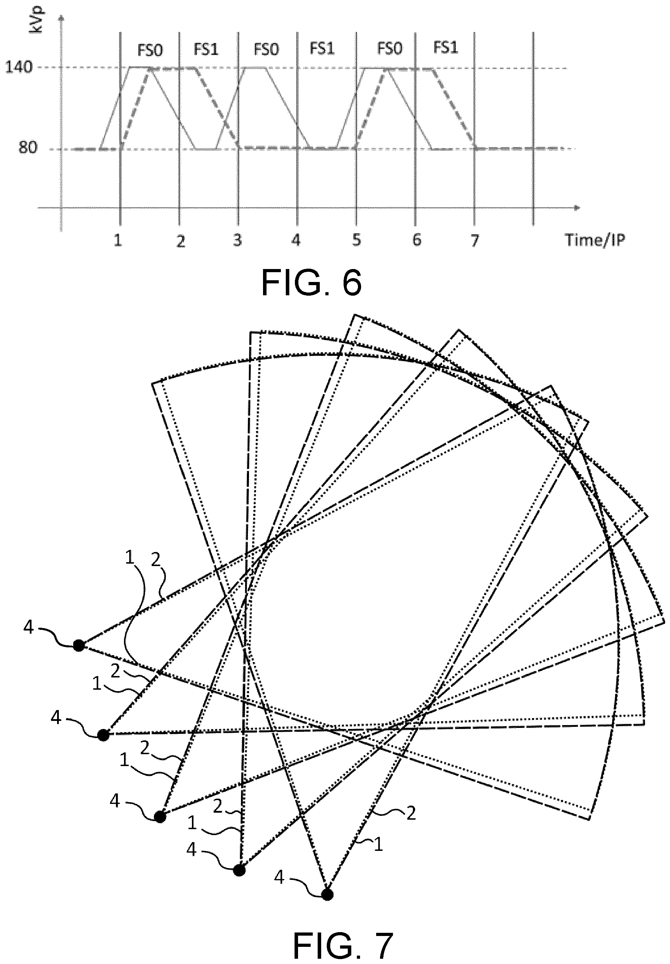

The image progression shows schematic images of the position of the x-ray source 3 on its way around the axis of rotation (roughly at the center of the indicated half circle). Each image corresponds to one integration period. Images that are in the same row show x-ray beams 1, 2 of with different energy spectra that have essentially the same path. This is achieved by the manipulation of the focal spot 4 which, on the images in the same lines, is at different positions with respect to the x-ray source's anode but at essentially the same position with respect to an absolute position relative the axis of rotation. Every second integration period, the peak kilovoltage is changed leading to x-ray beams 1, 2 with different x-ray spectra (represented by dotted and dashed lines, respectively). In this embodiment, there are two different focal spot configurations which are indicated by the relative position of the focal spot 4 relative to the schematically indicated x-ray source 3. The focal spot configurations are alternated for each image, corresponding to an alternating of the focal spot configuration each integration period. This alternation is indicated in Fig. 6, which shows the essentially the same course of the tube voltage as Fig. 4 and indicates an alternating between two focal spot configurations (FS0 and FS1) for each integration period. Generally, there may optionally be more focal spot configurations. For example, in case of a continuously rotating gantry 10, the focal spot configurations may be adapted continuously.

Based on the embodiment shown in Fig. 5, there are pairs of x-ray beams 1, 2 that temporarily follow directly one after the other and have different x-ray spectra corresponding to the first peak kilovoltage (dotted x-ray beams 1) and the second peak kilovoltage (dashed x-ray beams 2). By compensating for the rotation of the x-ray source during consecutive integration periods with different focal spot configurations, the pairs of x-ray beams 1, 2 cover essentially the same path, spatially.

Fig. 7 shows a schematic representation of adapting the focal spot configuration to achieve identical or very similar x-ray beam paths for different x-ray spectra, wherein several consecutively applied beams are shown at the same time. This Figure corresponds to Fig. 6 and may be regarded to show the individual images of Fig. 6 above each other. The positions of focal spots 4 are indicated which correspond to the position of the focal spot when generating the respective x-ray beam 1, 2. The positions of the focal spots correspond to the circular path of the x-ray source. It can be seen that there are pairs of x-ray beams 1, 2, of different spectra, corresponding to the first peak kilovoltage (dotted beams 1) and the second peak kilovoltage (dashed beams 2) that share essentially the same path. Corresponding to the pairs of x-ray beams 1, 2 there are pairs of focal spots 4 that have the same absolute position. Hence, the focal spot configuration is adapted via the location of the focal spots to compensate for the rotation of the gantry 10 between the generation of the pairs of x-ray beams 1, 2. Having the same pathways of the pairs of beams with different x-ray spectra can improve the process of material decomposition.

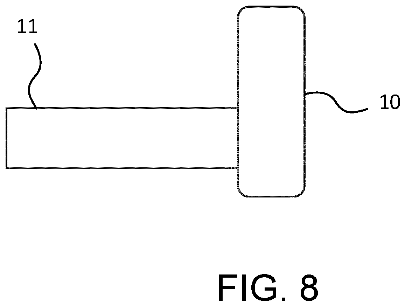

Fig. 8 shows a simplified side-view representation of a computed tomography system according to an embodiment of the invention. The computed tomography system comprises a gantry 10 and a patient table 11. The computed tomography system is configured to perform a method as described with respect to Fig. 2.

REFERENCE SIGNS

1     x-ray beam generated via the first peak kilovoltage

2     x-ray beam generated via the second peak kilovoltage

3     focal spot

4     x-ray source

10    gantry

11    patient table

**Claims**

1.  A method for performing spectral computed tomography imaging with a computed tomography system comprising a rotating gantry (10) with an x-ray source (4) and an x-ray detector, the method comprising the following steps:

    (a) consecutively operating the x-ray source (4) at a first peak kilovoltage over a time frame covering at least part of a first integration period of the x-ray detector and at least part of a second integration period of the x-ray detector;
    (b) switching the output of the x-ray source (4) from the first peak kilovoltage to a second peak kilovoltage, the second peak kilovoltage being different in peak height from the first peak kilovoltage;
    (c) consecutively operating the x-ray source (4) at the second peak kilovoltage over a time frame covering at least part of a third integration period of the x-ray detector and at least part of a fourth integration period of the x-ray detector;
    (d) switching the output of the x-ray source (4) from the second peak kilovoltage to the first peak kilovoltage;
    (e) periodically continuing the pattern of steps (a) to (d) for at least some integration periods;

    wherein, during each integration period, a focal spot configuration is applied to the x-ray source (4), such that, spatially, the x-ray beam (1) in the first integration period at least partially overlaps with an x-ray beam (2) of a previous integration period, the x-ray beam of the second integration (1) period at least partially overlaps with the x-ray beam (2) of the third integration period, and the x-ray beam of the fourth (2) integration period at least partially overlaps with the x-ray beam (1) of a next integration period.

**2.** The method according to claim 1,
wherein the focal spot configuration in the third integration period differs from the focal spot configuration in the second integration period such that, spatially, the x-ray beam (2) in the third integration period and the x-ray beam (1) in the second integration period are rotationally shifted towards each other, in particular at least diminishing an angular separation of the two beams that is due to the gantry's (10) rotation.

**3.** The method according to claim 2,
wherein the difference between the second focal spot configuration and the third focal spot configuration is such that the angular separation of the two beams that is due to the x-ray source's (4) rotation is essentially compensated.

**4.** The method according to any one of the preceding claims,

wherein the second peak kilovoltage is lower than the first peak kilovoltage,
wherein switching the output of the x-ray source (4) from the first peak kilovoltage to the second peak kilovoltage is performed within the second integration period and switching the output of the x-ray source (4) from the second peak kilovoltage to the first peak kilovoltage is performed in the integration period following after the fourth integration period.

**5.** The method according to claim 4,
wherein the second peak kilovoltage is applied continuously essentially over the entirety of the third integration period and the fourth integration period.

**6.** The method according to any one of the preceding claims,
wherein the focal spot configuration during the integration periods is controlled by manipulating the electron beam that is used for the x-ray generation of the x-ray source (4), in particular via electrostatic and/or magnetic fields.

**7.** The method according to claim 6,
wherein the spot at which the electron beam hits the anode of the x-ray source (4) is controlled to be different for the focal spot configurations when operating the x-ray source (4) at the first peak kilovoltage than for the focal spot configurations when operating the x-ray source (4) at the second peak kilovoltage during two integration periods following directly one after the other.

**8.** The method according to any one of the preceding claims,
wherein the x-ray detector comprises multiple angularly arranged detector elements, wherein the angular shift between the focal spot configuration of consecutive integration periods is set to correspond to the angular distance or a multiple of the angular distance between the angular position of neighboring detector elements of the detector.

**9.** The method according to any one of the preceding claims,
wherein a ratio between the first peak kilovoltage and the second peak voltage is at least 1.2, preferably at least 1.5, more preferably at least 1,7 and/or wherein a ratio between first peak kilovoltage and the second peak voltage is at most 2.5, preferably at most 2.2, more preferably at most 2.

**10.** The method according to any one of the preceding claims,
wherein the first peak kilovoltage is in the range of 110 kVp to 1000 kVp, preferably 120 kVp to 200 kVp, more preferably 130 kVp to 150 kVp and/or wherein the second peak kilovoltage is in the range of 30 kVp to 100 kVp, preferably 50 kVp to 95 kVp, more preferably 70 kVp to 90 kVp.

**11.** The method according to any one of the preceding claims,
wherein the lengths of the integration periods are each in the range of 20 $\mu$s to 1000 $\mu$s, preferably 30 $\mu$s to 500 $\mu$s, more preferably 40 $\mu$s to 200 $\mu$s.

**12.** A computer program comprising instructions which, when the program is executed by a control station of a computed tomography system, cause the computed tomography system to carry out the method according to any one of the preceding claims.

**13.** A computed tomography system comprising a rotatable gantry (10) with an x-ray source (4) and an x-ray detector and a control station,
wherein the control station is configured to let the computed tomography system perform the method according to any one of claims 1 to 11.

**14.** A use of a flying focal spot technology in kilovoltage peak switching computed tomography.

**15.** The use according to claim 14, wherein the flying focal spot technology is used to create and/or increase a spatial overlap of at least two consecutively generated x-ray beams (1, 2) with different peak kilovoltage.

FIG. 1

FIG. 2

FIG. 3

FIG. 4

FIG. 5

FIG. 6

FIG. 7

FIG. 8

Europäisches Patentamt

European Patent Office

Office européen des brevets

# EUROPEAN SEARCH REPORT

**Application Number**

EP 24 16 7840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/163530 A1 (SAINATH PAAVANA [US] ET AL) 28 June 2012 (2012-06-28) * paragraphs [0049], [0050], [0051], [0055], [0061], [0085], [0098]; figures 9,12 * | 1-15 | INV. A61B6/03 A61B6/40 A61B6/00 |
| Y | US 2016/081636 A1 (KREMER FRANS HENK [NL] ET AL) 24 March 2016 (2016-03-24) * paragraphs [0085], [0086], [0098]; figures 1,5,6 * | 1-15 | |
| A | US 2023/000456 A1 (NAKANISHI SATORU [JP]) 5 January 2023 (2023-01-05) * paragraph [0149] - paragraph [0195]; figure 8 * | 1-15 | |
| A | YIFAN DENG ET AL: "Multi-energy blended CBCT spectral imaging and scatter-decoupled material decomposition using a spectral modulator with flying focal spot (SMFFS)", MEDICAL PHYSICS, AIP, MELVILLE, NY, US, vol. 51, no. 4, 13 March 2024 (2024-03-13) , pages 2398-2412, XP072614060, ISSN: 0094-2405, DOI: 10.1002/MP.17022 * the whole document * | 1-15 | **TECHNICAL FIELDS SEARCHED (IPC)**<br><br>A61B |
| A | HAO ZHOU ET AL: "Fast KV-Switching and Dual-Layer Flat-Panel Detector Enabled Cone-Beam CT Joint Spectral Imaging", ARXIV.ORG, CORNELL UNIVERSITY LIBRARY, 201 OLIN LIBRARY CORNELL UNIVERSITY ITHACA, NY 14853, 2 January 2024 (2024-01-02), XP091670201, * the whole document * | 1-15 | |

The present search report has been drawn up for all claims

| Place of search | Date of completion of the search | Examiner |
|---|---|---|
| Munich | 13 September 2024 | Marzal-Abarca, X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 24 16 7840

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2024

| Patent document cited in search report | Publication date | Patent family member(s) | Publication date |
|---|---|---|---|
| US 2012163530 A1 | 28-06-2012 | DE 102011056641 A1 | 28-06-2012 |
| | | JP 6014323 B2 | 25-10-2016 |
| | | JP 2012130687 A | 12-07-2012 |
| | | US 2012163530 A1 | 28-06-2012 |
| US 2016081636 A1 | 24-03-2016 | CN 105307573 A | 03-02-2016 |
| | | EP 2994052 A1 | 16-03-2016 |
| | | JP 5961775 B2 | 02-08-2016 |
| | | JP 2016516558 A | 09-06-2016 |
| | | US 2016081636 A1 | 24-03-2016 |
| | | WO 2014180809 A1 | 13-11-2014 |
| US 2023000456 A1 | 05-01-2023 | JP 2023006563 A | 18-01-2023 |
| | | US 2023000456 A1 | 05-01-2023 |

EPO FORM P0459

For more details about this annex : see Official Journal of the European Patent Office, No. 12/82